# EUROPEAN PATENT SPECIFICATION

(11) **EP 4 433 022 B1**
(45) Date of publication and mention of the grant of the patent: **18.06.2025**
(21) Application number: 22823623.8
(22) Date of filing: 18.11.2022
(51) Int. Cl.: A61K 8/42, A61K 8/68, A61Q 19/08

(54) **LIQUID COMPOSITION OF CERAMIDES AND PALMITIC ACID AMIDES**
FLÜSSIGE ZUSAMMENSETZUNG VON CERAMIDEN UND PALMITINSÄUREAMIDEN
COMPOSITION LIQUIDE DE CÉRAMIDES ET D'AMIDES D'ACIDE PALMITIQUE

(30) Priority: 18.11.2021 IT 202100029171
(43) Date of publication of application: 25.09.2024
(73) Proprietor: Unifarco S.p.A., 32035 Santa Giustina (BL) (IT)
(72) Inventor: BARATTO, Giovanni, 32035 Santa Giustina BL (IT)
(74) Representative: Perani & Partners S.p.A.
(86) International application number: PCT/IB2022/061150
(87) International publication number: WO 2023/089557

(56) References cited:
- EP-B1- 3 125 865
- JP-B2- 5 432 470
- DATABASE GNPD [online] MINTEL; 20 January 2020 (2020-01-20), ANONYMOUS: "VECUA Grand Night Cream", XP055926288, retrieved from https://www.gnpd.com/sinatra/recordpage/7199719/ Database accession no. 7199719
- DATABASE GNPD [online] MINTEL; 20 January 2020 (2020-01-20), ANONYMOUS: "VECUA Grand Night Cream", XP055926288, retrieved from https://www.gnpd.com/sinatra/recordpage/7199719/ Database accession no. 7199719
- "Complete in Gel Cream Enrich", GNPD, MINTEL, 1 January 2017 (2017-01-01), XP002781544
- SEMENZATO A ET AL: "A New Synthetic Endocannabinoid as Anti-Inflammaging Cosmetic Active: an In Vitro Study on a Reconstructed Skin Model", vol. 2019, no. 1, 3 January 2019 (2019-01-03), XP055936765, Retrieved from the Internet <URL:https://www.vitroscreen.com/WEBVS/wp-content/uploads/2019/02/2018-ARTICLE-ANTI-INFLAMMAGING-ACTIVE-ON-RHE_CUDP-100003.pdf>
- ANONYMOUS: "Cleansing Emulsion - Ceramage - Unifarco Biomedical", 28 July 2018 (2018-07-28), XP055936792, Retrieved from the Internet <URL:https://web.archive.org/web/20180728112046/https://www.unifarcobiomedical.com/solutions/anti-inflamm-aging/ceramage/cleansing-emulsion/> [retrieved on 20220629]

## Description

### FIELD OF THE INVENTION

The present invention relates to an optimized liquid composition based on ceramides and palmitic acid amides and related stable and effective topical formulations in preventing and counteracting the skin inflammatory processes underlying skin diseases and/or *inflamm-aging.*

### BACKGROUND ART

Skin aging is an extremely heterogeneous physiological process.

In fact, numerous molecular mechanisms and pathways leading to cellular senescence can be identified.

In recent years, numerous studies on longevity have highlighted the role of chronic low-grade inflammation in aging, calling this phenomenon *inflamm-aging* [Baylis D, Bartlett DB, Patel HP, Roberts HC. Understanding how we age: insights into inflammaging. Longev Healthspan. 2013 May 2;2(1); Minciullo, P.L., Catalano, A., Mandraffino, G. et al. Inflammaging and Anti-inflammaging: The Role of Cytokines in Extreme Longevity Arch. Immunol. Ther. Exp. 64, 111-126 (2076*).*]

According to recent scientific evidence, *inflamm-aging* is mainly involved in the development of neurological and cardiovascular diseases but can affect the cells of all organs, including the skin.

*Inflamm-aging* is established and spread at the cellular level and is a silent chronic inflammatory state that occurs when cells lose their ability in regulating inflammatory and defence processes from internal and external aggressions.

The causes for inflamm-aging are:
- internal: increase in free radicals due to physical and mental stress, unbalanced diet, chronic intake of drugs and hormonal therapies;
- external: UV rays, environmental pollution, lifestyle.

The consequences are an accelerated onset of the typical signs of skin aging:
- dryness;
- spots or discolorations;
- wrinkles;
- loss of tone and elasticity;
- redness;
- scaling;
- opacity;
- burning sensation or skin tightness

In addition to the ongoing inflammatory process, the skin, in particular sensitive skin, has a poor defence capacity, which is why it is physiologically more fragile and subject to alterations of the epidermal barrier. This is why it is more likely to develop and self-fuel *inflamm-aging* processes. The reinforcement of the barrier with a consequent reduction of inflammation and therefore of the release of pro-inflammatory cytokines can be another mechanism to be evaluated to combat skin aging.

The use of ceramide-based products helps in counteracting skin aging as it acts effectively on the skin by restructuring and protecting the epidermal barrier.

At the state of the art there are numerous cosmetic ingredients that are effective in actively counteracting the physiological process of skin aging. The term endocannabinoids refers to a heterogeneous family of amides and esters of polyunsaturated fatty acids found in various human tissues. Arachidonic acid ethanolamide (Anandamide, ANA) and 2-arachidonylglycerol are certainly the two most described derivatives in the literature *[*Sugiura T, Kobayashi Y, Oka S, Waku K. Biosynthesis and degradation of anandamide and 2-arachidonoylglycerol and their possible physiological significance. Prostaglandins Leukot Essent Fatty Acids 66: 173-192]

These endogenous derivatives bind with different affinities to the known cannabinoid receptors CB1 and CB2 and the effect of ANA has been shown to be dependent on its extracellular concentration, which is controlled in addition to the enzymatic system responsible for its synthesis, also by an intracellular transport system and an enzymatic degradation system (Fatty Acid Hydrolases, FAAH).

N-isopropyl- palmitoyl amide and palmitoylethanolamide are endocannabinoid-like molecules that can play a key role in balancing important inflammatory mediators and *inflammatory-aging.*

Phytosteryl/Octyldodecyl Lauroyl Glutamate (INCI) of formula IUPAC (di-(phytosteryl-2-octyldodecyl) -N-lauroyl-L-glutamate) is an active ingredient for the formulation of products for topical use, and is an eudermic oil with a chemical structure similar to the components of the epidermal barrier, in particular to intercellular lipids.

Formulations of topical products (e.g. cosmetics or medical devices) containing ceramides and/or endocannabinoid-like derivatives may be very effective in counteracting the inflammatory processes underlying altered barrier physiology. However, they are all poorly soluble ingredients and are known to have stability and crystallization concerns.

The formulation of cosmetics with poorly soluble and very unstable active ingredients therefore poses technical challenges to formulators, such as the instability of the ingredient, dispersion problems, quality and safety.

The main problem consists in the solubilisation of this raw material in the final formulation, requiring careful and continuous control in the production phases of industrial scale-up.

To overcome the problem, it is possible to act on the production method, varying the order of the stages of addition of raw materials; however, the heating process at high temperatures, normally necessary to dissolve waxes, for example, could cause the degradation of other raw materials that make up the formula. A good compromise may be the pre-solution with a solvent.

However, the selection of the right solvent that leads to the formulation of a product for safe, stable and effective topical use requires careful evaluation, on the part of the formulator, of several factors:
- solvent behaviour in the base (vehicle)
- tolerability after application (also linked to the peculiar characteristics of the application site). (For example, tissue inflamed with dermatitis)
- compliance by the end user. (The formulated product must be pleasant and reused several times).

JP5432470B2 discloses an oily composition containing ceramide dissolved in a mixture of a triglyceride of a fatty acid and a phytosteryl derivative.

EP 3125865 discloses a composition comprising ceramide and palmitic acid amide with monoethylamine dispersed as such in an oil-in-water emulsion together with other active ingredients and/or excipients.

In the online MINTEL GNPD database dated 20 January 2020 XP055926288 database accession no. 7199719 and dated 1 January 2017 XP002781544, two Vecua creams and night cream comprising a plethora of components including various types of ceramide ceramide NP and cetyl PG hydroxyethylpalmitamide are disclosed.

Semenzato et al in "A New Endocannabinoid as Anti-Inflammatory Cosmetic Active: An In vitro study on Reconstructed Skin Model" CURR. UPDATES DERMATOL. PROBL. Vol. 2019, number 1, 3 January 2019.XP055036765 disclose that a new cannabinoid, the isopalmide, on a reconstructed skin model exhibits anti-inflammatory properties.

On the Unifarco Biomedical website, a Ceramage^{®} branded day cream containing among the several elements ceramide, isopalmide and (di-(phytosteryl-2-octyldodecyl)-N-lauroyl-L-glutamate) is disclosed.

Ceramide and isopalmide (di-(phytosteryl-2-octyldodecyl) -N-lauroyl-L-glutamate) are dispersed in the commercial formulation directly and not in the form of a liquid composition.

### SUMMARY OF THE INVENTION

The applicant has now surprisingly found that both ceramides and palmitic acid amides can be dissolved in a single step by mixing with the Phytosteryl/Octyldodecyl Lauroyl Glutamate (INCI) of formula IUPAC (di-(phytosteryl-2-octyldodecyl)-N-lauroyl-L-glutamate), which is able to fully solubilise the two components and thus contributes to creating a stable liquid composition suitable for inclusion in compositions for topical use.

The object of the present invention is therefore a liquid composition consisting of:
a) at least one ceramide
b) at least one palmitic acid amide of formula (I)

   **CH₃ (CH₂)₁₄CONHR¹** (I)

   and
c) di-(phytosteryl-2-octyldodecyl)-N-lauroyl-L-glutamate wherein R¹ is a straight or branched alkyl selected from the group consisting of a C1-C6 straight or branched alkyl optionally substituted with a hydroxyl group, a C4-C6 cycloalkyl, and wherein said components (a) and (b) are dissolved in said component (c)

### DESCRIPTION OF THE FIGURES

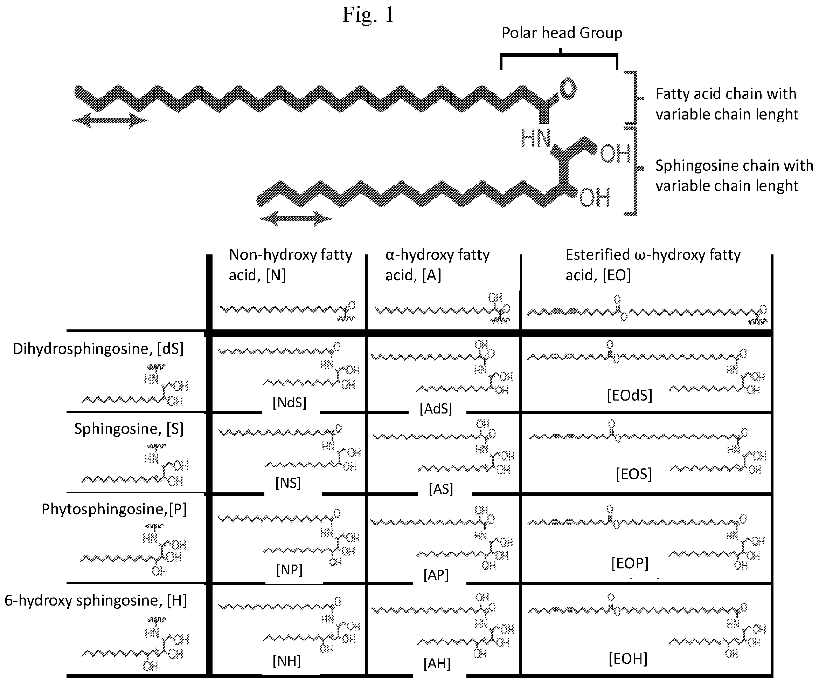

FIGURE 1 depicts the chemical formulas of some of the ceramides that may be used in the composition according to the present invention.

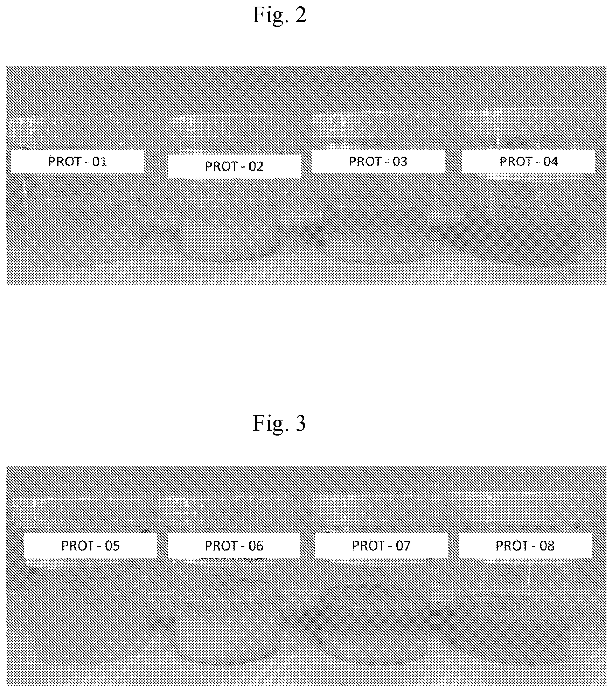

FIGURE 2 shows the photos of the solubilisation tests of 0.3% Ceramide 3 in different oils.
FIGURE 3 shows the photos of the solubilisation tests of 0.3% N-isopropyl- palmitoyl amide (Isopalmide) in different oils.

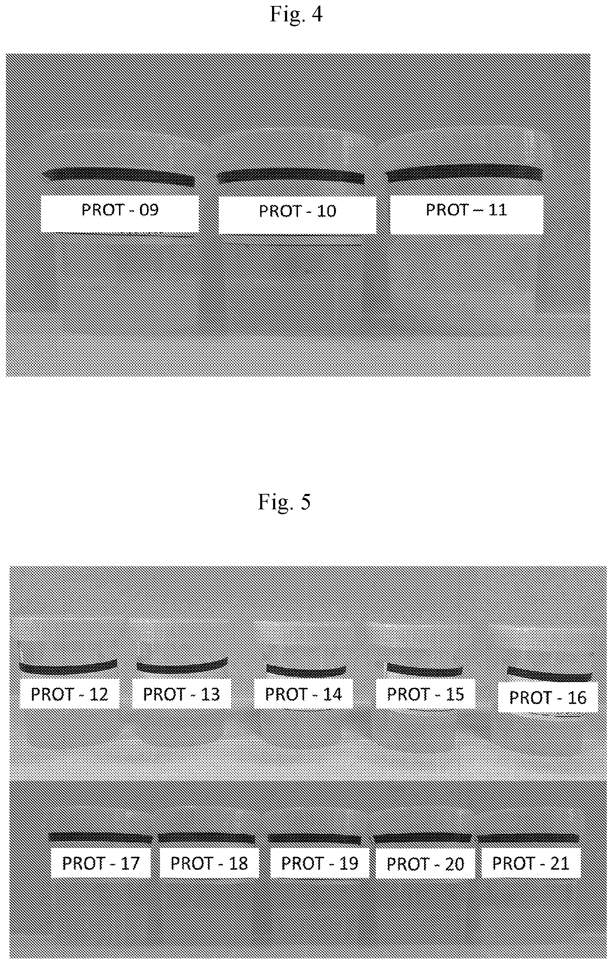

FIGURE 4 shows the photos of the solubilisation tests of 0.3% Palmitoylethanolamide (Palmitamide MEA or PMEA) in different oils.
FIGURE 5 shows the photos of the solubilisation tests of the liquid composition subject-matter of the patent, at increasing concentrations of N-isopropyl- palmitoyl amide (Isopalmide) and Ceramide 3.

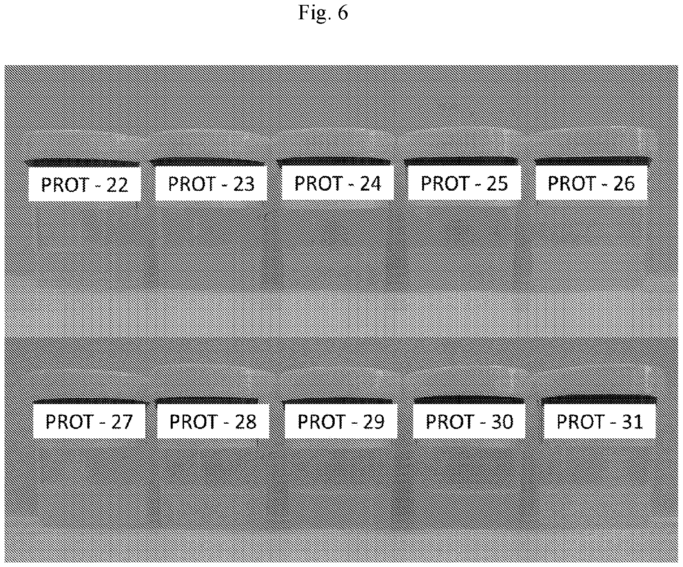

FIGURE 6 shows the photos of the solubilisation tests of the liquid composition subject-matter of the patent, at increasing concentrations of Palmitoylethanolamide (PMEA) and Ceramide 3.

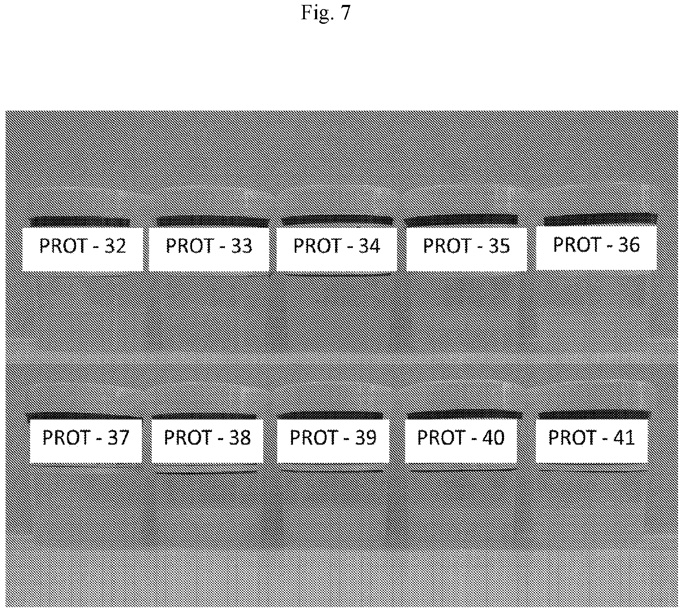

FIGURE 7 shows the photos of the solubilisation tests of the liquid composition subject-matter of the patent, at increasing concentrations of N-isopropyl- palmitoyl amide (Isopalmide), Palmitoylethanolamide (PMEA) and Ceramide 3.
Figures 8 show photos of the mixture of isopalmide, ceramide and di-(phytosteryl-2-octyldodecyl) -N-lauroyl-L-glutamate in the weight ratios between them in the Ceramage cosmetic formulations.

### DETAILED DESCRIPTION OF THE INVENTION

For the purposes of the present invention, the definitions "comprising" and "containing" provide for the possibility of further components in addition to those mentioned after said definition.

Conversely, for the purposes of the present invention the definition "consisting of" excludes the possibility of further components other than those listed after said definition.

In the present invention there is described a liquid composition consisting of:
(a) at least one ceramide,
(b) at least one palmitic acid amide of formula (I)

   **CH₃ (CH₂)₁₄CONHR¹** **(I)**
(c) di-(phytosteryl-2-octyldodecyl)-N-lauroyl-L-glutamate wherein said components (a) and (b) are dissolved

For the purposes of the present invention it is specified that, in the palmitic acid amide of formula (I), R¹ is a linear or branched alkyl selected from the group consisting of a C1-C6 linear or branched alkyl optionally substituted with a hydroxyl group, a C4-C6 cycloalkyl. In particular, R¹ is selected from: n-propyl, isopropyl, n-butyl, -n-pentyl, iso-pentyl, ethylene-hydroxy, cyclobutyl, cyclopentyl and cyclohexyl.

Preferably the palmitic acid amides of formula (I) are selected from N-isopropyl-palmitoyl amide and palmitoylethanolamide or a mixture thereof.

In the liquid composition subj ect-matter of the present invention the ceramide or component a) is selected from N-acetylfingosine (ceramide 2 or NS) or the amide of phytosphingosine with stearic acid (Ceramide 3 or NP), preferably it is the amide of phytosphingosine with stearic acid.

For the purposes of the present invention the weight ratio of the components a) and b) is comprised between 1:2 and 2:1, preferably said weight ratio of the components (a) and (b) is 1:1.

Preferably when in the liquid composition subject-matter of the present invention the component b) contains both the isopalmide and the PMEA in a weight ratio between 1:2 and 2:1 more preferably 1:1.

For the purposes of the present invention the components a) and b) are present at concentrations less than or equal to 7%, preferably comprised between 0.05 and 6%, more preferably between 0.05 and 2%, still more preferably between 0.1 and 1% by weight on the total weight of said association.

The liquid composition described in the present invention can be inserted into topical use formulations wherein said liquid composition is contained in amounts comprised between 0.5 and 10% more preferably between 1 and 5% by weight on the total weight of said topical formulation.

The topical formulation comprising the liquid composition subject-matter of the present invention may be in the form of an oil-in-water emulsion and in this case is substantially hydrophilic, or may also be a water-in-oil emulsion and thus be substantially lipophilic, or an anhydrous formulation thus lipophilic.

Topical formulations comprising the liquid composition described in the present invention can be used in treating and preventing skin inflammatory processes, in particular those related to *inflammation-aging.*

By skin inflammatory process, the Applicant also refers to the one developed by and/or associated with skin diseases such as: Atopic dermatitis (DIC, DAC), psoriasis, seborrhoeic dermatitis, acne, rosacea, actinic keratosis and ichthyosis.

Below is the procedure followed by the Applicant which allowed him to achieve at the present invention.

### 1- SELECTION OF THE SOLUBILISING AGENT: PROTOTYPE PREPARATION

The samples were made in the standard quantity of 50 g, sufficient to perform the solubilisation and stability tests. The concentration ranges of the investigated actives and the sample preparation methods were established after a critical reading of the technical data sheets of each raw material. The initial purpose of this work was to identify a solubilising agent for some raw materials such as Ceramide, N-isopropyl- palmitoyl amide (isopalmide) and palmitoylethanolamide (Palmitamide MEA or PMEA) that in the daily practice of a formulator exhibit problems of solubilisation.

To evaluate the stability over time of the prepared samples, the centrifuge test was performed, which is a method that allows to detect any instability effects such as sedimentation and phase separation following a condition of strong mechanical stress.

Table 1 shows the names of each prototype with the relative concentration of Ceramide, Isopalmide and PMEA and the different raw materials used initially as screening.

**Table 1 Systematic for the choice of the solubilising agent**

| PROTOTYP E NAME | ACTIV E % by weight | Solubilising Oil (INCI name) | Oper. solubilis. cond. | Appearance after solubilisatio n | appearanc e after 1 day | Post centrifuge appearanc e |
|---|---|---|---|---|---|---|
| PROT - 01 | ceramide -3 0.3 % | Squalane | Heat. up to 110 °C | solution, translucent solution | suspension and opaque solution | separated |
| PROT - 02 | ceramide -3 0.3 % | Phytosteryl macadamate/ squalane 50/50 (w/w) | Heat. up to 110 °C | opaque but homogeneou s solution | separated opaque solution with deposit | separated |
| PROT - 03 | ceramide -3 0.3 % | Phytosteryl/Octyldodec yl Lauroyl Glutamate / Phytosteryl /macadamate =50:50 w/w | Heat. up to 110 °C | clear transparent yellow solution | separated solution | separated |
| PROT - 04 | ceramide -3 0.3 % | Phytosteryl/Octyldodec yl Lauroyl Glutamate | Heat. up to 110 °C | clear yellow solution | opaque yellow solution | opaque yellow solution |
| PROT - 05 | Isopalmide | Squalane | Heat. up to 70°C | transparent solution | opaque solution | separated |
| | 0.3 % | | | | | |
| PROT - 06 | Iso palmide | Phytosteryl macadamate/ squalane = 50/50 w/w | Heat. up to 70 °C | transparent solution | separated solution with deposit | separated |
| | 0.3 % | | | | | |
| PROT - 07 | Isopalmide 0.3% | Phytosteryl/Octyldodec yl Lauroyl Glutamate: Phytosteryl macadamiate = 50:50 (w/w) | Heat. up to 70 °C | yellow transparent clear solution | separated solution with deposit | separated |
| PROT - 08 | Isopalmide 0.3% | Phytosteryl/Octyldodec yl Lauroyl Glutamate | Heat. up to 70°C | clear yellow solution | clear light yellow solution | clear light yellow solution |
| PROT - 09 | PMEA 0.3% | Squalane | Heat. up to 110 °C | transparent solution | opaque yellow solution | deposit on the bottom |
| PROT - 10 | PMEA 0.3% | Phytosteryl/Octyldodec yl Lauroyl Glutamate | Heat. up to 110 °C | transparent sol. | very clear yellow solution | very clear yellow solution |
| PROT - 11 | PMEA 0.3% | Phytosteryl macadamiate | Heat. up to 110 °C | transparent solution | opaque yellow solution | separated |

As can be observed by just using Phytosteryl/Octyldodecyl Lauroyl Glutamate as solubilising agent it is possible to obtain the individual solubilisation of Ceramide 3, isopalmide and PMEA.

### 2- SYSTEMATICS OF ANALYSES OF THE MIXTURES

After having unexpectedly identified that it was possible to use a single solubilising agent common to all the raw materials under study (ceramides and palmitic acid amides), systematics were set up to analyse the behaviour of the solution containing combinations of ceramides and palmitic acid amides, varying both the combination and the concentration of each component of the liquid composition subject-matter of the patent.

**Table 2 Systematics of Ceramide 3 with Isopalmide at increasing concentrations, solubilised in Phytosteryl/Octyldodecyl Lauroyl Glutamate**

| **PROTOT YPE NAME** | **Solubilising Oil (INCI name)** | **ceramid e -3** | **isopalmid e** | **Oper. Solubilisa tion cond.** | **appearance after solubilisation** | **appearan ce after 1 day** | **Post centrifuge appearanc e** |
|---|---|---|---|---|---|---|---|
| PROT - 12 | Phytosteryl/Oct yldodecyl Lauroyl Glutamate | 0.10% | 0.10 % | Heat. up to 110 °C | transparent solution | opaque yellow solution | opaque yellow solution |
| PROT - 13 | Phytosteryl/Oct yldodecyl Lauroyl Glutamate | 0.20% | 0.20 % | heating up to 110 °C | clear yellow solution | opaque yellow solution | opaque yellow solution |
| PROT - 14 | Phytosteryl/Oct yldodecyl Lauroyl Glutamate | 0.30% | 0.30 % | heating up to 110 °C | clear yellow solution | opaque yellow solution | opaque yellow solution |
| PROT - 15 | Phytosteryl/Oct yldodecyl Lauroyl Glutamate | 0.40 % | 0.40 % | Heat. up to 110 °C | clear yellow solution | opaque yellow solution | opaque yellow solution |
| PROT - 16 | Phytosteryl/Oct yldodecyl Lauroyl Glutamate | 0.50% | 0.50% | Heat. up to 110 °C | clear yellow solution | opaque yellow solution | opaque yellow solution |
| PROT - 17 | Phytosteryl/Oct yldodecyl Lauroyl Glutamate | 0.60% | 0.60% | Heat. up to 110 °C | clear yellow solution | opaque yellow solution | opaque yellow solution |
| PROT - 18 | Phytosteryl/Oct yldodecyl Lauroyl Glutamate | 0.70% | 0.70% | Heat. up to 110 °C | clear yellow solution | opaque yellow solution | opaque yellow solution |
| PROT - 19 | Phytosteryl/Oct yldodecyl Lauroyl Glutamate | 0.80% | 0.80% | Heat. up to 110 °C | clear yellow solution | opaque yellow solution | opaque yellow solution |
| PROT - 20 | Phytosteryl/Oct yldodecyl Lauroyl Glutamate | 0.90% | 0.90% | Heat. up to 110 °C | clear yellow solution | opaque yellow solution | opaque yellow solution |
| PROT - 21 | Phytosteryl/Oct yldodecyl Lauroyl Glutamate | 1.00 % | 1.00 % | Heat. up to 110 °C | clear yellow solution | opaque yellow solution | opaque yellow solution |

**Table 3 Systematics of Ceramide 3 with PMEA at increasing concentration, solubilised in Phytosteryl/Octyldodecyl Lauroyl Glutamate**

| **PROTOTY PE NAME** | **Solubilising Oil (INCI name)** | **cerami de -3** | **pme a** | **observatio ns** | **appearance after solubilisati on** | **appearan ce after 1 day** | **Post centrifuge appearan ce** |
|---|---|---|---|---|---|---|---|
| PROT - 22 | Phytosteryl/Octyldod ecyl Lauroyl Glutamate | 0.10 % | 0.10 % | Heat. up to 110 °C | transparent solution | semi-transp. solution yellow | semi-transp. solution yellow |
| PROT - 23 | Phytosteryl/Octyldod ecyl Lauroyl Glutamate | 0.20 % | 0.20 % | Heat. up to 110 °C | transparent solution | yellow opaque solution | yellow opaque solution |
| PROT - 24 | Phytosteryl/Octyldod ecyl Lauroyl Glutamate | 0.30 % | 0.30 % | Heat. up to 110 °C | transparent solution | yellow opaque solution | yellow opaque solution |
| PROT - 25 | Phytosteryl/Octyldod ecyl Lauroyl Glutamate | 0.40 % | 0.40 % | Heat. up to 110 °C | transparent solution | yellow opaque solution | yellow opaque solution |
| PROT - 26 | Phytosteryl/Octyldod ecyl Lauroyl Glutamate | 0.50% | 0.50 % | Heat. up to 110 °C | transparent solution | yellow opaque solution | yellow opaque solution |
| PROT - 27 | Phytosteryl/Octyldod ecyl Lauroyl Glutamate | 0.60% | 0.60 % | Heat. up to 110 °C | transparent solution | yellow opaque solution | yellow opaque solution |
| PROT - 28 | Phytosteryl/Octyldod ecyl Lauroyl Glutamate | 0.70% | 0.70 % | Heat. up to 110 °C | transparent solution | yellow opaque solution | yellow opaque solution |
| PROT - 29 | Phytosteryl/Octyldod ecyl Lauroyl Glutamate | 0.80% | 0.80 % | Heat. up to 110 °C | transparent solution | yellow opaque solution | yellow opaque solution |
| PROT - 30 | Phytosteryl/Octyldod ecyl Lauroyl Glutamate | 0.90% | 0.90 % | Heat. up to 110 °C | transparent solution | yellow opaque solution | yellow opaque solution |
| PROT - 31 | Phytosteryl/Octyldod ecyl Lauroyl Glutamate | 1.00 % | 1.00 % | Heat. up to 110 °C | transparent solution | yellow opaque solution | yellow opaque solution |

**Table 4 Systematics of Ceramide 3 Isopalmide and PMEA at increasing concentration, solubilised in Phytosteryl/Octyldodecyl Lauroyl Glutamate**

| **PROTOT YPE NAME** | **Solubilising Oil (INCI name)** | **cerami de -3** | **isopalm ide** | **pmea** | **Oper. Solubilisa tion cond.** | **appearan ce after solubilisa tion** | **appeara nce after 1 day** | **Post centrifu ge appeara nce** |
|---|---|---|---|---|---|---|---|---|
| PROT - 32 | Phytosteryl/Octyld odecyl Lauroyl Glutamate | 0.10% | 0.10 % | 0.10 % | Heat. up to 110 °C | transparen t solution | semi-transpare nt yellow solution | semi-transpare nt yellow solution |
| PROT - 33 | Phytosteryl/Octyld odecyl Lauroyl Glutamate | 0.20% | 0.20 % | 0.20 % | Heat. up to 110 °C | transparen t solution | opaque yellow solution | opaque yellow solution |
| PROT - 34 | Phytosteryl/Octyld odecyl Lauroyl Glutamate | 0.30% | 0.30 % | 0.30 % | Heat. up to 110 °C | transparen t solution | opaque yellow solution | opaque yellow solution |
| PROT - 35 | Phytosteryl/Octyld odecyl Lauroyl Glutamate | 0.40 % | 0.40 % | 0.40 % | heating up to 110 °C | transparen t solution | opaque yellow solution | opaque yellow solution |
| PROT - 36 | Phytosteryl/Octyld odecyl Lauroyl Glutamate | 0.50% | 0.50% | 0.50 % | Heat. up to 110 °C | transparen t solution | opaque yellow solution | opaque yellow solution |
| PROT - 37 | Phytosteryl/Octyld odecyl Lauroyl Glutamate | 0.60% | 0.60% | 0.60 % | Heat. up to 110 °C | transparen t solution | opaque yellow solution | opaque yellow solution |
| PROT - 38 | Phytosteryl/Octyld odecyl Lauroyl Glutamate | 0.70% | 0.70% | 0.70 % | Heat. up to 110 °C | transparen t solution | opaque yellow solution | opaque yellow solution |
| PROT - 39 | Phytosteryl/Octyld odecyl Lauroyl Glutamate | 0.80% | 0.80% | 0.80 % | Heat. up to 110 °C | transparen t solution | opaque yellow solution | opaque yellow solution |
| PROT - 40 | Phytosteryl/Octyld odecyl Lauroyl Glutamate | 0.90% | 0.90% | 0.90 % | Heat. up to 110 °C | transparen t solution | opaque yellow solution | opaque yellow solution |
| PROT - 41 | Phytosteryl/Octyld odecyl Lauroyl Glutamate | 1.00 % | 1.00 % | 1.00 % | Heat. up to 110 °C | transparen t solution | opaque yellow solution | opaque yellow solution |

For illustrative purposes, the following cosmetic formulations are reported in which the liquid compositions according to the present invention.

### Formulation 1 O/W EMULSION

| **Component** | **range [% w/w]** |
|---|---|
| Aqua | as needed up to 100 |
| Oils (vegetable or synthetic), Waxes, Butters (C10-18 triglycerides, Butyrospermum parkii butter, Alba wax, Caprylic/Capric triglyceride) | 5-30 |
| Silicones (Dimethicone, Dimethicone crosspolymer, dimethiconol) | 1-10 |
| Wetting agents (Glycerin, propylene glycol, butylene glycol) | 1-10 |
| Rheology modifiers (Polyacrylate crosspolymer-6, Carbomer, xanthan gum) | 1-5 |
| Emulsifiers (C14-22 Alcohols, C12-20 Alkyl Glucoside, PEG-100 stearate, potassium cethyl phosphate) | 0.5-5 |
| Preservatives (Potassium sorbate, sodium benzoate, phenoxyethanol) | 0.1-1.5 |
| Stabilizers (chelators, antioxidants) (disodium EDTA, Pentaerythrityl tetra-di-t-butyl hydroxyhydrocinnamate, tocopherol) | 0.1-2 |
| Perfume | 0.1-1 |
| **liquid composition of prot-21** | **0.5-10%** |

### Formulation 2 -SERUM

| **Component** | **range [% w/w]** |
|---|---|
| Aqua | as needed up to 100 |
| Oils (vegetable or synthetic), Waxes, Butters (C10-18 triglycerides, Butyrospermum parkii butter, Alba wax, Caprylic/Capric triglyceride) | 3-15 |
| Silicones (Dimethicone, Dimethicone crosspolymer, dimethiconol) | 1-5 |
| Wetting agents (Glycerin, propylene glycol, butylene glycol) | 1-10 |
| Rheology modifiers (Polyacrylate crosspolymer-6, Carbomer, xanthan gum) | 1-5 |
| Emulsifiers (C14-22 Alcohols, C12-20 Alkyl Glucoside, PEG-100 stearate, potassium cethyl phosphate) | 0.5-5 |
| Preservatives (Potassium sorbate, sodium benzoate, phenoxyethanol) | 0.1-1.5 |
| Stabilizers (chelators, antioxidants) (disodium EDTA, Pentaerythrityl tetra-di-t-butyl hydroxyhydrocinnamate, tocopherol) | 0.1-2 |
| Perfume | 0.1-1 |
| **liquid composition as prot-21** | **0.5-10%** |

### Formulation 3 - Detergent

| **Component** | **range [% w/w]** |
|---|---|
| Aqua | as needed up to 100 |
| Oils (vegetable or synthetic), Waxes, Butters (C10-18 triglycerides, Butyrospermum parkii butter, Alba wax, Caprylic/Capric triglyceride) | 3-15 |
| Wetting agents (Glycerin, propylene glycol, butylene glycol) | 1-10 |
| Rheology modifiers (Polyacrylate crosspolymer-6, Carbomer, xanthan gum) | 1-5 |
| Surfactants (Lauryl glucoside, Laureth-7 citrate, Sodium lauroyl sarcosinate) | 0.5-5 |
| Preservatives (Potassium sorbate, sodium benzoate, phenoxyethanol) | 0.1-1.5 |
| Stabilizers (chelators, antioxidants) (disodium EDTA, Pentaerythrityl tetra-di-t-butyl hydroxyhydrocinnamate, tocopherol) | 0.1-2 |
| Perfume | 0.1-1 |
| **liquid composition as prot-21** | **0.5-10%** |

### Formulation 4 O/W EMULSION

Formulation 4 contains the same components and in the same amounts as formulation 1 with the only difference being that the liquid composition of PROT.31 is used in the same amounts as the liquid composition of PROT.21 used in formulation 1.

### Formulation 5 SERUM

Formulation 5 contains the same components and in the same amounts as formulation 2 with the only difference being that the liquid composition of PROT.31 is used in the same amounts as the liquid composition 21 of the serum of formulation 2.

### Formulation 6 DETERGENT

Formulation 6 contains the same components and in the same amounts as formulation 3 with the only difference being that the liquid composition of PROT.31 is used in the same amounts as the liquid composition PROT.21 of the serum of formulation 3.

### Formulation 7 O/W EMULSION

Formulation 7 contains the same components and in the same amounts as formulation 1 with the only difference being that the liquid composition of PROT.40 is used in the same amounts as the liquid composition prot.21 used in formulation 1.

### Formulation 8 SERUM

Formulation 8 contains the same components and in the same amounts as formulation 2 with the only difference being that the liquid composition of PROT.40 is used in the same amounts as the liquid composition PROT 21 of the serum of formulation 2.

### Formulation 9 DETERGENT

Formulation 9 contains the same components and in the same amounts as formulation 3 with the only difference being that the liquid composition of PROT.40 is used in the same amounts as the liquid composition PROT.21 of the serum of formulation 3.

### Formulation 10 O/W EMULSION

Formulation 10 contains the same components and in the same amounts as formulation 1 with the only difference being that the liquid composition of PROT.41 is used in the same amounts as the liquid composition Prot.21 used in formulation 1.

### Formulation 11 SERUM

The formulation 11 contains the same components and in the same amounts as the formulation 2 with the only difference being that the liquid composition PROT.41 is used in the same amounts as the liquid composition PROT.21 of the serum of the formulation 2.

### Formulation 12 DETERGENT

Formulation 12 contains the same components and in the same amounts as formulation 3 with the only difference being that the liquid composition of PROT.41 is used in the same amounts as the liquid composition PROT.21 of the serum of formulation 3.

### COMPARATIVE TEST with Ceramage.

As anticipated in the state of the art of the present disclosure, the commercial product Ceramage produced by the owner of the present patent application contains the same components as the liquid composition as claimed in claim 1, however, at the concentrations used in Ceramage these cannot give rise to liquid compositions.

In fact, in the Ceramage product the concentrations of components a), b) and c) are as follows
Ceramide 0.3%, isopalmide 0.5% and di-(phytosteryl-2-octyldodecyl)-N-lauroyl-L-glutamate 0.5%.

A mixture of 100g of the above compounds was then prepared which respected the weight ratios of the three compounds in Ceramage.

So the content in this mixture of ceramide was 23g, that of isopalmide, as well as that of di-(phytosteryl-2-octyldodecyl) -N-lauroyl-L-glutamate was 38.5g each.

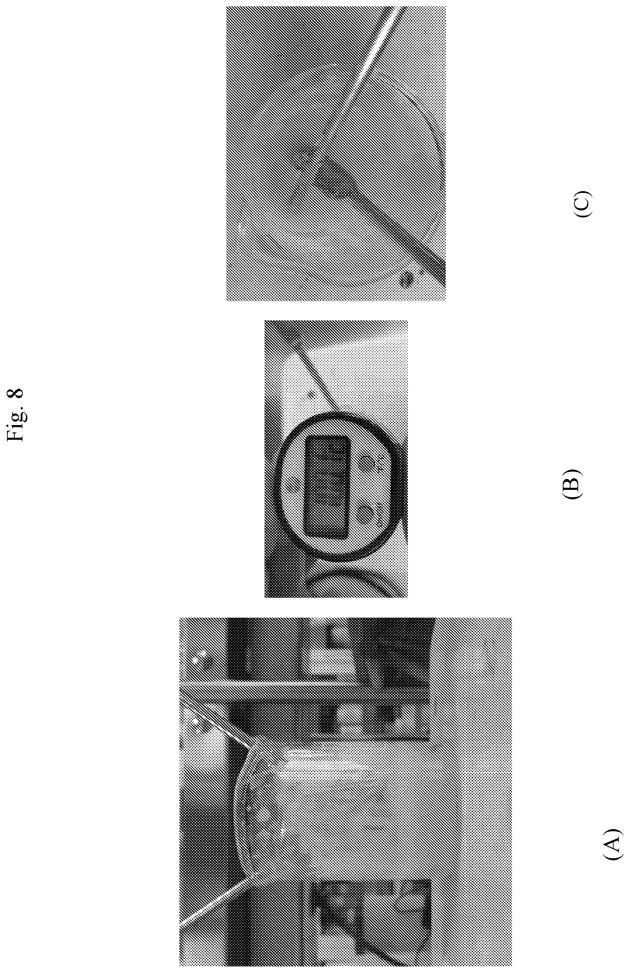

At such high concentrations of isopalmide as well as of ceramide compared to di-(phytosteryl-2-octyldodecyl) -N-lauroyl-L-glutamate, it is not possible to obtain liquid compositions of the aforementioned components, in fact the mixture of these three components, even if kept under magnetic stirring at 100°C, gives rise to a heterogeneous suspension (see figures 8(A)and 8(B)), which once the magnetic stirring is suspended and the temperature is lowered by a few degrees, already tends to solidify (Fig. 8(C)).

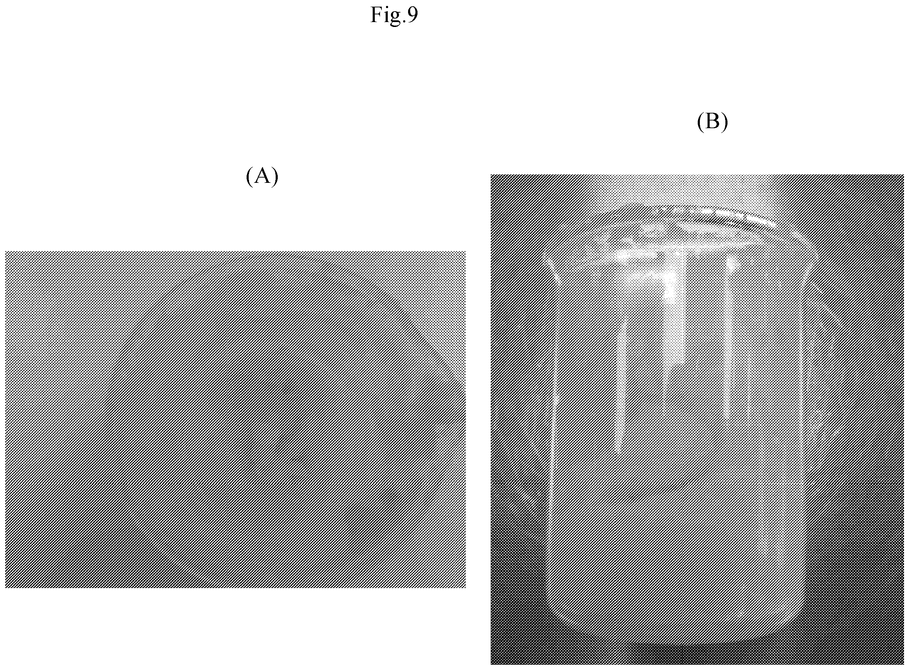

After 48 hours at room temperature, the mixture is completely solidified as shown in Figure 9(A) and 9(B) in the latter photo air bubbles are visible within the solidified mixture together with crystal formations.

## Claims

1. **Liquid composition** consisting of:
a) at least one ceramide
b) at least one palmitic acid amide of formula (I)
**CH₃ (CH₂)₁₄CONHR¹** **(I)**
and
c) di-(phytosteryl-2-octyldodecyl)-N-lauroyl-L-glutamate wherein R¹ is a straight or branched alkyl selected from the group consisting of a C1-C6 straight or branched alkyl optionally substituted with a hydroxyl group, a C4-C6 cycloalkyl, and wherein said components a) and b) are dissolved in said component c).

2. Liquid composition according to claim 1, wherein R¹ is selected from: n-propyl, isopropyl, n-butyl, -n-pentyl, iso-pentyl, ethylene-hydroxy, cyclobutyl, cyclopentyl and cyclohexyl.

3. Liquid composition according to claim 1 or 2, wherein the palmitic acid amides are selected from N-isopropyl- palmitoyl amide and palmitoylethanolamide or a mixture thereof.

4. Liquid composition according to any one of claims 1-3, wherein said ceramide or component a) is selected from N-acetylsphyingosine (ceramide 2 or NS) or the amide of phytosphingosine with stearic acid (Ceramide 3 or NP), preferably it is the amide of phytosphingosine with stearic acid.

5. Liquid composition according to any one of claims 1-4, wherein each component a) and b) is present in concentrations comprised between 0.05 and 2% by weight on the total weight of said association, preferably between 0.1 and 1% by weight.

6. Liquid composition according to any one of claims 1-5, wherein the weight ratio of the components a) and b) is comprised between 1:2 and 2:1.

7. Liquid composition according to any one of claims 1-6, wherein the weight ratio of the components a) and b) is 1:1.

8. Liquid composition according to any one of claims 3-7, wherein in component b) both the N-isopropylpalmitoyl amide and the palmitoylethanolamide are present in weight ratios comprised between 1:2 and 2:1, preferably 1:1.

9. **Topical formulation** comprising the liquid composition according to any one of claims 1-8 in amounts comprised between 0.5 and 10% by weight on the total weight of said topical formulation.

10. Topical formulations according to claim 9, wherein said liquid composition is contained in amounts between 1 and 5% by weight, on the total weight of said topical formulation.

11. Topical formulation according to claim 9 or 10, in the form of an oil-in-water emulsion and is substantially hydrophilic.

12. Topical formulation according to claim 9 or 10, in the form of a water-in-oil emulsion and is substantially lipophilic.

13. Topical formulation according to claim 9 or 10, in anhydrous form.

14. **Topical formulation** according to any one of claims 9-13 **for use** in the treatment and prevention of skin inflammatory processes.

15. Topical formulation for use according to claim 14, wherein said skin inflammatory processes are associated or involved in skin inflammatory diseases or are associated or involved in skin ageing processes.

## Patentansprüche

1. **Flüssige Zusammensetzung** bestehend aus:
a) mindestens einem Ceramid
b) mindestens einem Palmitinsäureamid von Formel (I)
**CH₃ (CH₂)₁₄CONHR¹** **(I)**
und
c) di-(Phytosteryl-2-octyldodecyl)-N-lauroyl-L-glutamat, wobei R¹ ein gerades oder verzweigtes Alkyl ist, das ausgewählt ist aus der Gruppe, bestehend aus einem geraden oder verzweigten C1-C6-Alkyl, optional substituiert mit einer Hydroxylgruppe, einem C4-C6-Cycloalkyl, und wobei die Komponenten a) und b) in der Komponente c) gelöst sind.

2. Flüssige Zusammensetzung nach Anspruch 1, wobei R¹ ausgewählt ist aus: n-Propyl, Isopropyl, n-Butyl, -n-Pentyl, Iso-Pentyl, Ethylen-Hydroxy, Cyclobutyl, Cyclopentyl und Cyclohexyl.

3. Flüssige Zusammensetzung nach Anspruch 1 oder 2, wobei die Palmitinsäureamide ausgewählt sind aus N-Isopropylpalmitoylamid und Palmitoylethanolamid oder einem Gemisch davon.

4. Topische Zusammensetzung nach einem der Ansprüche 1-3, wobei das Ceramid oder die Komponente a) ausgewählt ist aus N-Acetylfingosin (Ceramid 2 oder NS) oder dem Amid von Phytosphingosin mit Stearinsäure (Ceramid 3 oder NP), es bevorzugt das Amid von Phytosphingosin mit Stearinsäure ist.

5. Topische Zusammensetzung nach einem der Ansprüche 1-4, wobei jede Komponente a), b), c) und jede Komponente d) in Konzentrationen zwischen 0,05 und 2 Gewichts-% des Gesamtgewichts der Zusammensetzung, vorzugsweise zwischen 0,1 und 1 Gewichts-%, vorhanden ist.

6. Flüssige Zusammensetzung nach einem der Ansprüche 1-5, wobei das Gewichtsverhältnis der Komponenten a) und b) zwischen 1:2 und 2: 1 liegt.

7. Flüssige Zusammensetzung nach einem der Ansprüche 1-6, wobei das Gewichtsverhältnis der Komponenten a) und b) 1:1 ist.

8. Flüssige Zusammensetzung nach einem der Ansprüche 3-7, wobei in Komponente b) sowohl das N-Isopropylpalmitoylamid als auch das Palmitoylethanolamid in Gewichtsverhältnissen zwischen 1:2 und 2: 1, vorzugsweise 1:1, vorhanden sind.

9. **Topische Formulierung,** umfassend die flüssige Zusammensetzung nach einem der Ansprüche 1-8 in Mengen zwischen 0,5 und 10 Gewichts-%, bezogen auf das Gesamtgewicht der topischen Formulierung.

10. Topische Formulierungen nach Anspruch 9, wobei die flüssige Zusammensetzung in Mengen zwischen 1 und 5 Gewichts-%, bezogen auf das Gesamtgewicht der topischen Formulierung, enthalten ist.

11. Topische Formulierung nach Anspruch 9 oder 10 in Form einer Öl-in-Wasser-Emulsion und die im Wesentlichen hydrophil ist.

12. Topische Formulierung nach Anspruch 9 oder 10 in Form einer Wasser-in-Öl-Emulsion und die im Wesentlichen lipophil ist.

13. Topische Formulierung nach Anspruch 9 oder 10 in anhydrischer Form.

14. **Topische Formulierung** nach einem der Ansprüche 9-13 **zur Verwendung** bei der Behandlung und Vorbeugung von hautentzündlichen Prozessen.

15. Topische Formulierung zur Verwendung nach Anspruch 14, wobei die hautentzündlichen Prozesse mit hautentzündlichen Erkrankungen assoziiert oder dabei involviert sind oder mit Alterungsprozessen der Haut assoziiert oder dabei involviert sind.

## Revendications

1. **Composition liquide** constituée de :
a) au moins un céramide
b) au moins un amide d'acide palmitique de formule (I)
**CH₃ (CH₂)₁₄CONHR¹** **(I)**
et
c) di-(phytostéryl-2-octyldodécyl)-N-lauroyl-L-glutamate dans laquelle R¹ est un alkyle linéaire ou ramifié choisi dans le groupe constitué d'un alkyle linéaire ou ramifié en C1-C6 éventuellement substitué par un groupe hydroxyle, un cycloalkyle en C4-C6, et dans laquelle lesdits composants a) et b) sont dissous dans ledit composant c).

2. Composition liquide selon la revendication 1, dans laquelle R¹ est choisi parmi : n-propyle, isopropyle, n-butyle, -n-pentyle, iso-pentyle, éthylène-hydroxy, cyclobutyle, cyclopentyle et cyclohexyle.

3. Composition liquide selon la revendication 1 ou 2, dans laquelle les amides d'acide palmitique sont choisis parmi le N-isopropyl-palmitoylamide et le palmitoyléthanolamide ou un mélange de ceux-ci.

4. Composition liquide selon l'une quelconque des revendications 1 à 3, dans laquelle ledit céramide ou composant a) est choisi parmi la N-acétylsphingosine (céramide 2 ou NS) ou l'amide de phytosphingosine avec acide stéarique (céramide 3 ou NP), de préférence il s'agit de l'amide de phytosphingosine avec acide stéarique.

5. Composition liquide selon l'une quelconque des revendications 1 à 4, dans laquelle chaque composant a) et b) est présent dans des concentrations comprises entre 0,05 et 2 % en poids par rapport au poids total de ladite association, de préférence entre 0,1 et 1 % en poids.

6. Composition liquide selon l'une quelconque des revendications 1 à 5, dans laquelle le rapport pondéral des composants a) et b) est compris entre 1:2 et 2: 1.

7. Composition liquide selon l'une quelconque des revendications 1 à 6, dans laquelle le rapport pondéral des composants a) et b) est de 1:1.

8. Composition liquide selon l'une quelconque des revendications 3 à 7, dans laquelle dans le composant b), le N-isopropylpalmitoyl amide et le palmitoyléthanolamide sont présents dans des rapports pondéraux compris entre 1:2 et 2:1, de préférence 1:1.

9. **Formulation topique** comprenant la composition liquide selon l'une quelconque des revendications 1 à 8 dans des quantités comprises entre 0,5 et 10 % en poids par rapport au poids total de ladite formulation topique.

10. Formulations topiques selon la revendication 9, dans lesquelles ladite composition liquide est contenue dans des quantités comprises entre 1 et 5 % en poids, par rapport au poids total de ladite formulation topique.

11. Formulation topique selon la revendication 9 ou 10, sous la forme d'une émulsion huile dans eau et est sensiblement hydrophile.

12. Formulation topique selon la revendication 9 ou 10, sous forme d'émulsion eau dans huile et est sensiblement lipophile.

13. Formulation topique selon la revendication 9 ou 10 sous forme anhydre.

14. **Formulation topique** selon l'une quelconque des revendications 9 à 13, **destinée à être utilisée** dans le traitement et la prévention de processus inflammatoires cutanés.

15. Formulation topique destinée à être utilisée selon la revendication 14, dans laquelle lesdits processus inflammatoires cutanés sont associés ou impliqués dans des maladies inflammatoires cutanées ou sont associés ou impliqués dans des processus de vieillissement cutané.
